# EUROPEAN PATENT APPLICATION

(11) **EP 1 167 528 A1**
(43) Date of publication of application: **02.01.2002**
(21) Application number: 00202207.7
(22) Date of filing: 23.06.2000
(51) Int. Cl.: C12N 15/53, C12N 9/02, C12N 1/21, C12N 5/10, C12P 15/00, A01N 63/04

(54) **Botrytis cinerea laccase**

(71) Applicant: Wageningen University, 6700 HB Wageningen (NL)
(72) Inventor: Schouten, Alexander, 6866 De Heelsum (NL); Van Kan, Johannes Arnoldus Laurentius, 3911 JP Rhenen (NL); Stefanato, Francesca Lucia, 6709 RN Wageningen (NL); Sibbel-Wagemakers, Cornelia Antonia Maria, 6708 NA Wageningen (NL)
(74) Representative: de Bruijn, Leendert C.

(57) **Abstract**

The present invention describes a protein having laccase activity. This protein is derived from *Botrytis cinerea,* which is a common fungal grapevine pathogen. The protein it was found to convert resveratrol into fungitoxic compounds. According to the invention the laccase can be used to protect plants against animal and microbial pests.

### Annex to the application documents - subsequently filed sequences listing

SEQUENCE LISTING

<110> Wageningen University

<120> Novel laccase

<130> Novel laccase

<140> 0020207.7
<141> 2000-06-23

<160> 4

<170> PatentIn Ver. 2.1

<210> 1
<211> 581
<212> PRT
<213> Botrytis cinerea

<220>
<221> METAL
<222> (125)..(127)
<223> Putative copper binding site

<220>
<221> METAL
<222> (169)..(171)
<223> Putative copper binding site

<220>
<221> METAL
<222> (467)..(469)
<223> Putative copper binding site

<220>
<221> METAL
<222> (526)..(528)
<223> Putative copper binding site

<220>
<221> SIGNAL
<222> (1)..(21)
<223> Putative signal sequence

<400> 1
Met Lys Tyr Ser Thr Val Phe Thr Ala Leu Thr Ala Leu Phe Ala Gln
  1               5                  10                  15

Ala Ser Ala Thr Ala Ile Pro Ala Val Arg Ser Pro Leu Ala Pro Arg
            20                  25                  30

Gln Ser Thr Thr Ala Ser Cys Ala Asn Ser Ala Thr Ser Arg Ser Cys
        35                  40                  45

Trp Gly Glu Tyr Ser Ile Asp Thr Asn Trp Tyr Asp Val Thr Pro Asn
    50                  55                  60

Thr Gly Val Thr Arg Glu Tyr Trp Leu Ser Val Glu Asn Ser Thr Ile
65                  70                  75                  80

Thr Pro Asp Gly Tyr Thr Arg Ser Ala Met Thr Phe Asn Gly Thr Val
                85                  90                  95

Pro Gly Pro Ala Ile Thr Ala Asp Trp Gly Asp Asn Leu Ile Ile His
            100                 105                 110

Val Thr Asn Asn Leu Gln His Asn Gly Thr Ser Ile His Trp His Gly
        115                 120                 125

Ile Arg Gln Leu Gly Ser Leu Glu Tyr Asp Gly Val Pro Gly Val Thr
    130                 135                 140

Gln Cys Pro Ile Ala Pro Gly Asp Thr Leu Thr Tyr Lys Phe Gln Ala
145                 150                 155                 160

Thr Gln Tyr Gly Thr Thr Trp Tyr His Ser His Phe Ser Leu Gln Tyr
                165                 170                 175

Ala Asp Gly Leu Phe Gly Pro Leu Ile Ile Asn Gly Pro Ala Thr Ala
            180                 185                 190

Asp Tyr Asp Glu Asp Val Gly Ala Ile Phe Leu Gln Asp Trp Ala His
        195                 200                 205

Lys Ser Val Phe Glu Ile Trp Asp Ser Ala Arg Gln Gly Ala Pro Pro
    210                 215                 220

Ala Leu Glu Asn Thr Leu Met Asn Gly Thr Asn Ile Tyr Asp Cys Ser
225                 230                 235                 240

Ala Ser Thr Asp Ala Asn Cys Val Gly Gly Gly Lys Lys Phe Glu Leu
                245                 250                 255

Thr Phe Val Glu Gly Thr Lys Tyr Arg Leu Arg Leu Ile Asn Val Gly
            260                 265                 270

Ile Asp Ser His Phe Glu Phe Ala Ile Asp Asn His Thr Leu Thr Val
        275                 280                 285

Ile Ala Asn Asp Leu Val Pro Ile Val Pro Tyr Thr Thr Asp Thr Leu
    290                 295                 300

Leu Ile Gly Ile Gly Gln Arg Tyr Asp Val Ile Val Glu Ala Asn Ala
305                 310                 315                 320

Ala Ala Asp Asn Tyr Trp Ile Arg Gly Asn Trp Gly Thr Thr Cys Ser
                325                 330                 335

Ser Asn Ser Glu Ala Ala Asn Ala Thr Gly Ile Leu Arg Tyr Asp Ser
            340                 345                 350

Ser Ser Thr Val Asp Pro Thr Ser Val Gly Val Thr Pro Arg Gly Thr
        355                 360                 365

Cys Ala Asp Glu Pro Val Ala Ser Leu Val Pro His Leu Ala Leu Asp
    370                 375                 380

Val Gly Gly Tyr Ser Leu Val Asp Glu Gln Val Ser Phe Ala Phe Thr
385                 390                 395                 400

Asn Tyr Phe Thr Trp Thr Ile Asn Ser Ser Ser Leu Leu Leu Asp Trp
                405                 410                 415

Ser Ser Pro Thr Thr Leu Lys Ile Phe Asn Asn Glu Thr Ile Phe Pro
            420                 425                 430

Thr Asp Tyr Asn Val Val Ala Leu Asn Gln Thr Asp Ala Asn Glu Glu
        435                 440                 445

Trp Val Val Tyr Val Ile Glu Asp Leu Thr Gly Phe Gly Ile Trp His
    450                 455                 460

Pro Ile His Leu His Gly His Asp Phe Tyr Val Val Ala Gln Glu Thr
465                 470                 475                 480

Asp Val Phe Ser Ala Thr Lys Ser Pro Ala Asn Phe Asn Leu Val Asn
                485                 490                 495

Pro Pro Arg Arg Asp Val Ala Ala Leu Pro Gly Asn Gly Tyr Leu Ala
            500                 505                 510

Ile Ala Phe Lys Leu Asp Asn Pro Gly Ser Trp Leu Leu His Cys His
        515                 520                 525

Ile Ala Trp His Ala Ser Glu Gly Leu Ala Met Gln Phe Val Glu Ser
    530                 535                 540

Gln Ser Ser Ile Ala Ile Gly Met Ser Asp Thr Asp Ile Phe Glu Asp
545                 550                 555                 560

Thr Cys Ala Asn Trp Asn Ala Tyr Thr Pro Thr Glu Leu Phe Ala Glu
                565                 570                 575

Asp Asp Ser Gly Ile
            580

<210> 2
<211> 21
<212> PRT
<213> Botrytis cinerea

<400> 2
Met Lys Tyr Ser Thr Val Phe Thr Ala Leu Thr Ala Leu Phe Ala Gln
  1               5                  10                  15

Ala Ser Ala Thr Ala
             20

<210> 3
<211> 35
<212> DNA
<213> Artificial Sequence

<220>
<223> Description of Artificial Sequence:Gene specific
      primer

<400> 3
catgaagtat tctacagtct ttactgccct cactg                                35

<210> 4
<211> 28
<212> DNA
<213> Artificial Sequence

<220>

<223> Description of Artificial Sequence:Gene specific
      primer

<400> 4
cccttagatt ccagaatcgt cctcggcg                                    28

## Description

### Background of the invention

*Botrytis cinerea* is a common fungal grapevine pathogen which causes significant losses for vineyard owners worldwide. It is mostly found in temperate regions and is known to cause grey mould. Next to grapevines (*Vitis vinifera*), *Botrytis cinerea* can infect a wide variety of other hosts such as fruits, flowers and green tissue of more than 200 plant species.

It is known that plants contain a general defense mechanism against infection by phytopathogenic fungi. This defense includes the production of a chemically divergent group of antimicrobial agents, called phytoalexins. The major component in grapevine phytoalexin response is resveratrol (*trans-*3, 5, 4'-trihydroxystilbene). This compound protects grapevines, but also other plants, against *Botrytis cinerea* by rapidly accumulating in healthy tissues surrounding the site of the *Botrytis cinerea* infection leading to slowing or halting the spread of the infection. Although elevated levels of resveratrol are fungitoxic against *Botrytis cinerea*, some highly pathogenic *Botrytis cinerea* strains can circumvent this defense mechanism by detoxifying resveratrol.

According to the state of the art (see Hoos and Blaich (1990) J. Phytopathology 129, 102-110; Pezet *et al.* (1991) Physiological and Molecular Plant Pathology 39, 441-450; Sbaghi *et al.* (1996) Plant Pathology 45, 139-144; Adrian *et al.* (1998) Phytopathology 88, 472-476; Breuil *et al.* (1999) Phytopathology 89, 298-301 and Cichewicz *et al.* (2000) J. Nat. Prod. 63, 29-33), resveratrol can be detoxified by *Botrytis cinerea* and other fungi through the action of a laccase. Laccases are multi-copper-containing enzymes that catalyze the oxidation of numerous phenolic substrates. Laccases are found in plants, bacteria and a wide variety of fungi including ascomycetes such as *Botrytis, Aspergillus, Neurospora* and *Podospora* and basidiomycetes such as *Collybia, Fomes, Lentinus, Pleurotus* and *Trametes.*

### Summary of the invention

The present invention relates to a novel enzyme having laccase activity. This enzyme has been found in *Botrytis cinerea* and the amino acid sequence has been determined. Surprisingly, this novel laccase does not detoxify resveratrol. On the contrary, it was found to convert resveratrol into fungitoxic compounds. The present invention thus pertains to a protein with laccase activity capable of converting resveratrol into fungitoxic compounds, to DNA encoding it and to vectors and host cells containing such DNA. Furthermore, the invention relates to the use of these vectors and host cells in producing the laccase protein and the fungitoxic compounds as described by the appending claims.

### Description of the invention

It was found according to the invention that fungitoxic compounds are produced from resveratrol by *Botrytis* strains, in particular by *Botrytis cinerea*, as a result of the activity of a novel laccase.

Two different complete laccase encoding sequences were found in a genomic library of *Botrytis cinerea*. The first laccase gene (*Bclcc*1), which is homologous to the partial DNA sequence used as a probe (Genbank accession No. U20192), comprises 1871 nucleotides. This sequence contains 3 introns and encodes a protein of 561 amino acids. The second laccase gene (*Bclcc*2; Genbank accession No. AF243855) is significantly different from *Bclcc*1. This novel gene consists of 2042 nucleotides. It contains 3 introns and encodes a protein of 581 amino acids depicted in SEQ ID No. 1. The deduced molecular weight of BcLCC2 is 63 kDa and its deduced isoelectric point is 4.32.

The present invention covers a protein having laccase activity with an amino acid identity of at least 60%, preferably at least 70%, and more preferably at least 80%, compared to the amino acid sequence of SEQ ID No. 1. The invention also covers a part of a protein with at least 15 contiguous amino acids which are identical to the corresponding part of the amino acid sequence of SEQ ID No. 1. The novel laccase has homology with several other proteins as revealed by amino acid sequence alignment. A high homology was found with BcLCC1 laccase of *Botrytis cinerea* (52.8% identity), Laccase 1 of *Cryphonectria parasitica* (Genbank accession No. Q03966; 34.9% identity), Laccase 1 of *Neurospora crassa* (Genbank accession No. P06811; 33.0% identity), Laccase 2 of *Neurospora crassa* (Genbank accession No. P10574; 33.3% identity) and Laccase 2 of *Podospora anserina* (Genbank accession No. P78722; 33.2% identity). The BcLCC2 protein contains 4 putative copper binding sites, which are characterized by the His-X-His motif, at the positions 125-127, 169-171, 467-469 and 525-528 of the amino acid sequence of SEQ ID No. 1. Furthermore, BcLCC2 contains a putative signal sequence MKYSTVFTALTA LFAQASATA at position 1-21 of the amino acid sequence of SEQ ID No. 1.

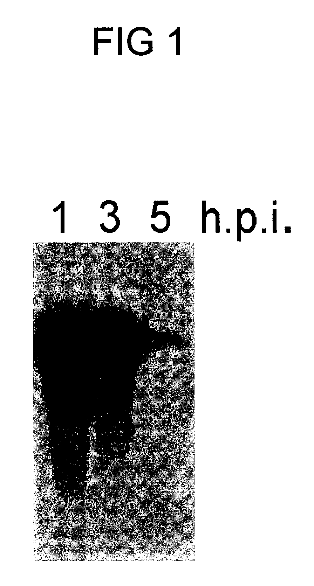

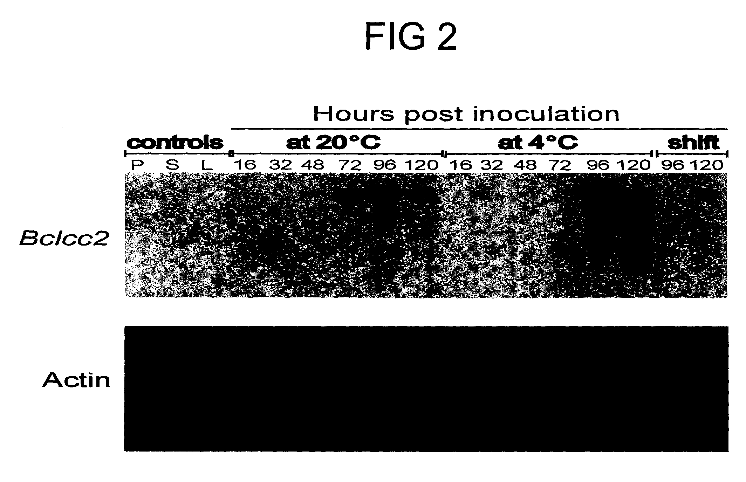

A nucleotide sequence encoding any of the above mentioned proteins, mutants, variants or parts thereof is also a subject of the invention. The above-mentioned nucleotide sequence can be present in the genomic form, i.e. including the introns, or in the form which corresponds to the cDNA. The nucleic acid sequences corresponding to expression-regulating regions (promoters, enhancers or terminators) located upstream or downstream the nucleotide sequence encoding the novel laccase with the amino acid sequence of SEQ ID No. 1 are also a part of the invention. These nucleotide sequences can be used for homologous or heterologous expression of genes. Such expression-regulating sequences are operationally linked to a polypeptide-encoding nucleic acid sequence such as the gene of the laccase according to the invention. It has been demonstrated by Northern blotting (see figure 1) that the transcription of *Bclcc*2 is inducible by substrates of the novel laccase such as tannic acid or resveratrol. Furthermore, during colonization of tomato leaves by *Botrytis cinerea*, it was found that *Bclcc2* is transcribed at high levels, relative to total fungal biomass, at 20°C starting at 48 h post infection (see figure 2). In general, at this time point the fungus starts to spread from its primary site of infection. During growth of the fungus in liquid medium, supplemented with polygalacturonic acid or sucrose, no *Bclcc*2 transcripts were discovered, suggesting a possible function of BcLCC2 during colonisation of host tissue.

A nucleic acid construct comprising a nucleotide sequence according to the invention operationally linked to an expression-regulating nucleic acid sequence is also covered by the invention. The nucleic acid construct is preferably a vector, in particular a plasmid, cosmid or phage. The choice of vector is dependent on the recombinant procedures followed and the host cell used. The vector may be an autonomously replicating vector or may replicate together with the chromosome into which it has been integrated. Preferably, the vector contains a selection marker. Useful markers are dependent on the host cell of choice and are well known to persons skilled in the art. The expression-regulating nucleic acid sequence such as a promoter, a ribosome binding site, a terminator, a translation initiation signal, a repressor gene or an activator gene can be any nucleic acid sequence showing activity in the host cell of choice and can be derived from genes encoding proteins, which are either homologous or heterologous to the host cell.

A recombinant host cell, such as a mammalian (with the exception of human), plant, animal, fungal yeast or bacterial cell, containing one or more copies of the nucleic acid construct mentioned above is an additional subject of the invention. Examples of suitable bacteria are Gram positive bacteria such as several *Bacillus* or *Streptomyces* strains or Gram negative bacteria such as *Escherichia coli.* For optimal expression (secretion into the culture medium) and protein maturation (folding) in bacteria it may be necessary to exchange the endogenous signal peptide coding sequence of BcLCC2 for a bacterial one such as the presequence of an amylase gene from a *Bacillus* species or to completely delete the endogenous signal peptide coding sequence of BcLCC2 and to introduce an alternative translational start codon. Additionally, the translation stop codon can be omitted and a c-myc and/or polyhistidine coding sequence can be added in frame C-terminally to facilitate protein purification and protein detection on western blot and in an ELISA. Expression in yeast can be achieved by using yeast strains such as *Pichia pastoris*, *Saccharomyces cerevisiae* and *Hansenula polymorpha.* For optimal expression (secretion into the culture medium) and protein maturation (folding) it may be necessary to exchange the endogenous signal peptide coding sequence of BcLCC2 for the yeast α-factor signal sequence or to completely delete the endogenous signal peptide coding sequence of BcLCC2 and to introduce an alternative translational start codon. Additionally, the translation stop codon can be omitted and replaced by a coding sequence as described for bacterial cells. Alternatively, a suitable expression system can be a baculovirus system or expression systems using mammalian cells such as CHO or COS cells. In a preferred embodiment of the invention the host cells are plant cells, preferably cells of crop plants such as forestry plants, for example spruce, fir, douglas fir, pine, larch, beech and oak or of plants providing food and raw materials such as cereal plants, in particular wheat, rye, barley, oats, millet, rice and corn, potatoes, legumes, in particular pulses and alfalfa, soya beans, vegetables, in particular brassicas and tomatoes, fruit, in particular apples, pears, cherries, citrus fruits, pineapples, bananas and more particularly grapes, oil palms, tea plants, cocoa and coffee plants, tobacco plants, sisal and cotton, of medicinal plants, in particular *Rauwolfia* and *Digitalis*, of ornamentals, in particular roses and gerberas, and of flower bulb crops, in particular tulip, iris, gladiolus, narcissus or hyacinth. Transformed (transgenic) plants or plant cells are produced by known methods, for example, by transformation of leaf discs (see for example, Horsch *et al*. (1985) Science 277, 1229-1231), by co-culture of regenerating plant protoplasts or cell cultures with *Agrobacterium tumefaciens* (see for example, Marton *et al.* (1979) Nature 277, 1229-1231 or Hain *et al*. (1985) Mol. and Gen. Genet. 199, 161-168) or by direct DNA transfection. Resulting transformed plants are identified either by selection for expression of the reporter gene, for example by phosphorylation of kanamycin sulphate (see Reiss *et al*. (1984) Gene 1081, 211-217 or Schreier *et al*. (1985) EMBO J. 4, 25-32), or by expression of nopaline synthase by the method of Aerts *et al*. (1983) Plant Sc. Lett. 17, 43-50 or of *Bclcc*2 expression by Northern and Western blot analysis. For optimal expression, the *Bclcc*2 cDNA gene can be placed under translational control of a truncated cauliflower mosaic virus (CaMV) Cabb B-D 35S promoter (-343/-1) with duplicated enhancer sequence (-343/-90) together with the 38 base pair alfalfa mosaic virus (AlMV) RNA4 untranslated leader (Sijmons *et al*. (1990) Biotechnology 8, 217-221). As a terminator the nopaline synthase terminator can be used. For optimal expression (secretion into the culture medium) and protein maturation (folding) it may be necessary to exchange the endogenous signal peptide coding sequence of *Bclcc*2 for a plant one. Additionally, the translation stop codon can be omitted and a c-myc and/or polyhistidine coding sequence can be added in frame C-terminally to facilitate protein purification and protein detection on western blot and in an ELISA.

The present invention also pertains to a process of producing a laccase as described above. The laccase may be isolated by conventional means from the culture of a laccase-positive *Botrytis* strain, especially *Botrytis cinerea*, or from a recombinant organism expressing the laccase (*Bclcc*2) gene. In *Botrytis* strains the laccase can be recovered from the culture medium and in recombinant host cells the protein can be recovered from the cell free extract, but preferably it is recovered from the culture medium.

A process of producing phytoalexins by converting resveratrol using the novel laccase protein according to the invention is also a part of the present invention. The phytoalexins can be produced *in vitro* by adding resveratrol to the purified laccase protein or by adding resveratrol to the culture medium of a *Botrytis* strain or by a recombinant host cell capable of producing and preferably secreting the novel laccase into the culture medium and recovering the produced phytoalexins from the culture medium. In a preferred embodiment of the invention the phytoalexins are produced *in vivo* by plant cells capable of producing both resveratrol and the novel laccase protein. In plant cells not containing the appropriate proteins necessary to synthesize resveratrol, the genes of these enzymes can be introduced additionally by methods known in the art (see US 5,985,647). In such an embodiment of the invention, the *Bclcc*2 cDNA is preferably placed under transcriptional control of the stilbene synthase (StiSy) promoter to synchronise resveratrol and BcLCC2 synthesis in a vector according to the invention. Substrates used by the stylbene synthase are malonyl-CoA and cinnamoyl-CoA or coumaroyl-CoA. These compounds are present in every plant, since they are also used in the biosynthesis of other important plant constituents such as flavonoids or flower pigments.

Finally, the use of a process of producing phytoalexins according to the above for controlling animal and microbial pests in plants or plant cells is also a part of the invention. Pests which may be mentioned against which, with the aid of the *Bclcc*2 gene according to the invention, resistances or increased resistances may be brought about, are animal pests, such as insects, mites and nematodes and microbial pests, such as bacteria and in particular phytopathogenic fungi. Noxious insects are in particular insects of the orders: *Orthoptera, Dermaptera, Isoptera, Thysanoptera, Heteroptera, Homoptera, Lepidoptera, Coleoptera, Hymenoptera* and *Diptera.* Noxious mites are in particular: *Tarsonemus spp*., *Panonychus spp.* and *Tetranychus spp.* Noxious nematodes are in particular: *Pratylenchus spp*., *Globodera spp*., *Heterodera spp.* and *Meloidogyne spp.* Microbial pests are in particular the phytopathogenic *Oomycetes* and fungi: *Plasmodiophoromycetes*, *Chytridiomycetes, Zygomycetes, Ascomycetes, Basidiomycetes* and *Deuteromycetes.* Phytopathogenic bacteria are in particular: *Pseudomonadaceae, Rhizobiaceae, Enterobacteriaceae, Corynebacteriaceae* and *Streptomycetaceae.* Some pathogens of fungal and bacterial diseases which are encompassed by the abovementioned generic terms and which may be mentioned as examples, but not by way of limitation, are: *Xanthomonas* species such as, for example, *Xanthomonas campestris pv. oryzae*; *Pseudomonas* species such as, for example, *Pseudomonas syringae pv. lachrymans; Erwinia* species such as, for example, *Erwinia amylovora; Pythium* species, such as, for example, *Pythium ultimum; Phytophthora* species such as, for example, *Phytophthora infestans; Pseudoperonospora* species such as, for example, *Pseudoperonospora humuli* or *Pseudoperonospora cubense; Plasmopara* species such as, for example, *Plasmopara viticola*; *Peronospora* species such as, for example, *Peronospora pisi* or *Peronospora brassicae*; *Erysiphe* species, such as, for example, *Erysiphe graminis*; *Sphaerotheca species* such as, for example, *Sphaerotheca fuliginea*; *Podosphaera* species such as, for example, *Podosphaera leucotricha*; *Venturia* species such as, for example, *Venturia inaequalis*; *Pyrenophora species* such as, for example, *Pyrenophora teres* or *Pyrenophora graminea*; *Cochliobolus* species such as, for example, *Cochliobolus sativus*; *Uromyces* species such as, for example, *Uromyces appendiculatus*; *Puccinia* species such as, for example, *Puccinia recondita*; *Tilletia* species such as, for example, *Tilletia caries; Ustilago* species such as, for example, *Ustilago nuda* or *Ustilago avenae*; *Pellicularia* species such as, for example, *Pellicularia sasakii*; *Pyricularia* species such as, for example, *Pyricularia oryzae*; *Fusarium* species such as, for example, *Fusarium colmorum*; *Botrytis* species such as, for example, *Botrytis cinerea*; *Septoria* species such as, for example, *Septoria nodorum*; *Leptosphaeria* species such as, for example, *Leptosphaeria nodorum*; *Cercospora* species such as, for example, *Cercospora canescens*; *Alternaria* species such as, for example, *Alternaria brassicae;* and *Pseudocercosporella* species such as, for example, *Pseudocercosporella herpotrichoides.*

The phytoalexins can either be produced by the plants themselves (as mentioned above) or *in vitro* according to the invention. When produced *in vitro* they can be obtained as emulsions, suspensions, solutions and distributed by spraying, over plants infected with animal or microbial pests or injected into soils.

### Description of the figures

Figure 1: Northern blot analysis of *Bclcc*2 expression in mycelium from a liquid culture of *Botrytis cinerea* wild type B05.10 1, 3 and 5 hours post induction (h.p.i.) with tannic acid.
Figure 2: The presence of *Bclcc*2 transcripts at different time points and temperatures during *Botrytis cinerea* colonization of tomato leaves. Equal amounts of total RNA isolated from infected leaves were loaded. The actin transcripts represent a measure of the total fungal RNA present in each sample. Controls: Total RNA isolated from fungal growth in liquid cultures supplemented with polygalacturonic acid (P) or sucrose (S) and from non-infected tomato leaves, *L. esculentum* (L). Shift: temperature shift from 4°C to 20°C 72 hrs h.p.i..
Figure 3: Construction of the pLCC1 transformation vector for homologous recombination.
Figure 4: The SalA and SalB fragment, both carrying part of the *Bclcc*2 gene were subcloned in pBluescript and used for the transformation vector pLCC2-HS.
Figure 5: *Botrytis cinerea* wild type B05.10, one *Bclcc*1 disruptant (Δ*Bclcc*1) and two individual *Bclcc*2 disruptants (Δ*Bclcc*2, *A* and B) grown on plates supplemented with tannic acid. The tannic acid conversion, resulting in a brownish precipitate, is absent for the two *Bclcc*2 disruptants. Circles indicate advance of the growing mycelium.
Figure 6: Wild type B05.10 and the *Bclcc*1 and *Bclcc*2 disruptants growing on plates containing 200 mg/ml resveratrol (dissolved in 0.2% methanol). Since methanol is the solvent of resveratrol a control plate containing 0.2% methanol was included.
Figure 7: Wild types B05.10 and SAS56 and the *Bclcc*1 and *Bclcc*2 disruptants growing on plates containing 200 mg/ml resveratrol (dissolved in 0.2% methanol).
Figure 8: Resveratrol conversion by culture filtrate obtained after mycelial growth of wild type strain B05.10 and a *Bclcc*2 disruptant 19 hours after induction with resveratrol, measured at 304 nm. The control represents non inoculated medium.

The invention is further illustrated by the following non-limiting examples.

### Examples

### Example 1: Isolation of the novel laccase gene (Bclcc2) from genomic DNA and Southern blotting.

The *Botrytis cinerea* wild type strain B05.10 described by Büttner *et al.* (1994) Current Genetics 25, 445-450, which is derived from the strain SAS56 described by van der Vlugt-Bergmans *et al.* (1993) Mycological Research 97, 1193-1200 was used for analysis, for transformation and as a wild type control strain for comparison. For long term storage the *Botrytis cinerea* strains were frozen at -80°C in 75% (v/v) glycerol containing 12 mM NaCl.

*Botrytis cinerea* was grown at 20°C in the dark on plates containing malt agar (Oxoid) or tPDA (potato dextrose agar (Oxoid) supplemented with 300 g homogenized tomato leaves per liter) or in liquid cultures by inoculating 100 ml Gamborg's B5 medium, supplemented with 10 mM sucrose and 10 mM NaH₂PO₄, with 10⁷ conidia. After 2-4 hours of preincubation, the germinating conidia were incubated for 24-48 hours, depending on the experiment, at 20°C in a rotary shaker at 180 rpm. Additionally, *Botrytis cinerea* was also grown on plates containing resveratrol. Therefore, Gamborg's B5 medium, supplemented with 10 mM sucrose and 10 mM NaH₂PO₄ and 15 g/l agar, was autoclaved and cooled to 50°C. Resveratrol (100 mg/ml), dissolved in methanol, was added to a final concentration of 0.2 mg/ml and the plates were poured immediately.

Plates completely covered with mycelium were used to isolate conidia. To induce sporulation these plates were placed under near UV light for 16 hours. Next conidia were harvested from the sporulating plates 7 to 14 days after induction using 5 ml sterile water, containing 0.05% (v/v) Tween 80. The suspension was filtered through glass wool, washed once by centrifugation at 114xg during 5 min and resuspended in sterile water.

*In vivo* expression of *Bclcc*1 and *Bclcc*2 laccase was investigated by a time course infection experiment on detached tomato leaves. Therefore, isolated *Botrytis cinerea* strain B05.10 conidia were preincubated for 2 hours in B5 medium supplemented with 10 mM glucose and 10 mM (NH₄)H₂PO₄ to stimulate germination. Leaves of tomato, *Lycopersicon esculentum* cultivar Moneymaker genotype *Cf* 4, were inoculated by spraying with the medium containing 10⁶ spores per ml. The leaves were incubated at 20°C in 95% humidity and subsequently harvested at regular intervals after inoculation and stored at -80°C.

*In vitro* induction of *Bclcc*2 transcription by tannic acid was performed as follows. 100ml Gamborg's B5 medium, supplemented with 10 mM sucrose and 10 mM NaH₂PO₄, was inoculated with 5×10⁷ conidia. After 2-4 hours of preincubation, the germinating conidia were incubated for 56 hours at 20°C in a rotary shaker at 180 rpm. The mycelium was harvested by filtration over Miracloth and transferred to 100 ml fresh B5 medium, supplemented with 10% mM sucrose, 10 mM NaH₂PO₄, 200 µM CuSO₄ and 125 mg/l tannic acid. Incubation was continued for 19 hours at 20°C in a rotary shaker at 180 rpm. The mycelium was harvested 1, 3 and 5 hours post induction by filtration over Miracloth, freeze dried and stored at -20°C until further analysis.

*In vitro* induction of BcLCC2 enzyme activity was measured after induction with either tannic acid or resveratrol. In case of tannic acid the following procedure was followed. 100ml Gamborg's B5 medium, supplemented with 10 mM sucrose and 10 mM NaH₂PO₄, was inoculated with 5×10⁷ conidia. After 2-4 hours of preincubation, the germinating conidia were incubated for 56 hours at 20°C in a rotary shaker at 180 rpm. The mycelium was harvested by filtration over Miracloth and transferred to 100 ml fresh B5 medium, supplemented with 10% (w/v) sucrose, 10 mM NaH₂PO₄, 200 µM CuSO₄ and 125 mg/l tannic acid (Sigma). Incubation was continued for 19 hours at 20°C in a rotary shaker at 180 rpm. The culture filtrate was separated from the mycelium by filtration over Miracloth and stored at -20°C until further analysis. In case of induction with resveratrol an identical procedure was used with the exception that after harvesting by filtration the mycelium was transferred to 100 ml fresh B5 medium, supplemented with 10% (w/v) sucrose, 10 mM NaH₂PO₄ and 50 µg/ml resveratrol.

BcLCC2 can be concentrated from culture medium by ultrafiltration over an BioMax PM30 Ultrafiltration disk with a molecular weight cutoff of 30 kDa (Amicon). Further purification of the protein can be achieved by gel chromatography and, when necessary, by ion exchange chromatography using standard procedures.

Laccase enzyme activity was detected spectrophotometrically using the syringaldazine assay or the resveratrol assay. In the syringaldazine assay to 850 µl 0.1 M citrate buffer, pH 5.4, 100 µl 0.5 mM syringaldazine (dissolved in ethanol) and 50 µl culture filtrate were added. After incubation for 30 min at room temperature the OD was measured at 525 nm. The resveratrol assay was modified from Pezet *et al.* (1991) Physiological and Molecular Plant Pathology 39, 441-450. To 1 ml 0.1 M citrate buffer, pH 5.2, 30 µl 0.5 ng/ml resveratrol (disolved in methanol) and 50 µl culture filtrate were added. The OD at 304 nm was measured at various time points.

The laccase gene was isolated from genomic DNA as follows. A genomic library of *Botrytis cinerea* strain SAS56 in lambda EMBL3 (see Van der Vlugt-Bergmans *et al*. (1997) Molecular Plant-Microbe Interactions 10, 21-29) was screened for the presence of laccase genes. As a probe a 1250 bp partial cDNA fragment encoding for a laccase (called Bclcc1) (Genbank accession No. U20192), which was derived from the same strain, was used. The individual hybridizing phages were isolated and phage DNA was isolated. From these phages hybridizing restriction fragments were subcloned in the pBluescript®SK(+) phagemid by procedures known in the art and subsequently the fragments were sequenced.

Genomic DNA was isolated by harvesting mycelium from a liquid culture by filtration over Miracloth (Calbiochem) and subsequent freeze drying. Next the dried mycelium was homogenized in liquid nitrogen by placing a pestle in the tube while vortexing. A solution containing 3 ml TES (100 mM Tris-HCl pH 8.0, 10 mM EDTA and 2% (w/v) SDS) and 60 µl proteinase K (20 µg/µl) was added and the suspension was incubated for one hour at 60°C. Subsequently, 840 µl 5 M NaCl and 130 µl 10% (w/v) N-cetyl-N,N,N-trimethylammonium bromide (CTAB) were added and incubation continued for 20 min at 65°C. Then the suspension was extracted by adding 4.2 ml chloroform/ isoamylalcohol (24:1 v/v) followed by vortexing shortly, incubation for 30 min on ice and centrifugation for 5 min at 18,000×g. The aqueous top phase was transferred and 1,350 µl 7.5 M NH₄Ac was added, it was incubated on ice for one hour and centrifuged for 15 min at 18,000×g. To precipitate the DNA, isopropanol was added to a final concentration of 41% (v/v). The DNA was transferred from the liquid using a glass rod washed in 70% (v/v) ethanol and dried. The genomic DNA was dissolved in 1 ml TE (10 mM Tris-HCl pH 7.5 and 0.1 mM EDTA) containing 2.5 U RNaseA, incubated for 30 min at 50°C and precipitated with ethanol. The pellet was dissolved in 200 µl TE. To estimate the concentration, 5 µl of this solution was loaded on a gel.

To investigate the presence of multiple copies of the novel laccase gene Southern blotting was performed. Therefore, 1 µg genomic DNA was digested to completion with 100 units of the desired restriction enzyme in a total volume of 100 µl. DNA fragments were separated on a 0.8% (w/v) agarose gel and subsequently blotted using the protocol for alkali blotting on Hybond™-N⁺ membrane (Amersham). Therefore, the gel containing the DNA was first placed in 0.25 M HCl until the dyes had changed color (this was only applied on the blots containing genomic DNA). After rinsing the gel in distilled water, a capillary blot was set up according to Sambrook *et al*. (1989) Molecular Cloning: A Laboratory Manual, second edition, Cold Spring Harbor Laboratory Press, Cold Spring Harbor, New York, USA, using 0.4 M NaOH as a blotting solution. After DNA transfer, the membrane was rinsed briefly in 2×SSC (0.3 M NaCl and 0.03 M sodium citrate, pH 7) and dried. The DNA was cross-linked to the membrane by UV treatment (312 nm, 0.6 J/cm²). The Random Primers DNA Labeling System (Life Technologies) was used to obtain by radioactively labeled probes. Therefore, 20 ng of the necessary DNA fragment was labeled according to the manufacturers' instructions and the labelled DNA fragments were purified on a Sephadex G50 column. Hybridisation was according to Church and Gilbert (1984) Proc. Natl. Acad. Sci. USA 81, 1991-1995. Briefly, the blot was prehybridized for 30 min at 65°C with hybridization buffer (0.25 M phospate buffer, pH 7.2, 1 mM EDTA, 1% (w/v) bovine serum albumin and 7% (w/v) SDS). Then, the blot was hybridized with 3 ml hybridization buffer, containing the labeled probe, for 40 hours at 65°C. The blots were washed three times for 30 min at 65°C in 2×SSC and 0.1% (w/v) SDS. Autoradiograms were made using Kodak X-OMAT AR film.

### Example 2: RNA isolation, Northern blotting, cDNA analysis.

Total RNA was isolated from *Botrytis cinerea* mycelium and infected leaves. Freeze dried mycelium was homogenized in liquid nitrogen by placing a pestle in the tube while vortexing. Leaf tissue was ground to powder in liquid nitrogen using mortar and pestle. Per gram material, 2 ml guanidine buffer pH 7.0 was added. This buffer consisted of 8.0 M guanidine hydrochloride, 20 mM 2-[N-morpholino]-ethanosulfonic acid (MES), 20 mM EDTA and 50 mM β-mercaptoethanol, pH 7.0. The suspension was extracted twice, once with an equal volume of phenol/chloroform/isoamylalcohol (IAA) (25:24:1 v/v/v) and once with chloroform/IAA (24:1 v/v). After subsequent centrifugation for 45 min at 12000xg at 4°C one-third volume of 8 M LiCI was added to the aqueous phase. Next, the suspension was incubated overnight on ice and centrifuged for 15 min at 12000xg. The pellet was washed once with 2 M LiCl and twice with 70% (v/v) ethanol, air-dried and dissolved in 1 ml TE. The concentration was determined spectrophotometrically at 260 nm. Alternatively, TRIzol™ Reagent (Life Technologies) was used according to the manufacturer's instructions to isolate total RNA from freeze-dried mycelium.

For gel electrophoresis of total RNA, samples were first denatured. Therefore, to 10 µg of total RNA in 3.7 µl, 3.6 µl 6 M deionised glyoxal, 10.7µl dimethyl sulfoxide and 2.0 µl 0.1 M sodium phosphate buffer, pH 7 was added. The sample was incubated at 50°C for 60 min, centrifuged briefly, chilled in liquid nitrogen and thawed again on ice. Next, 2 µl 10× loading buffer (50% (v/v) glycerol, 0.01 M sodium phosphate, 0.3% (v/v) bromophenolblue and 0.3% (v/v) xylene cyanol) was added prior to loading on a 1.4% (w/v) agarose gel. Gel and running buffer contained 0.01 M phospate buffer, pH 7. After gel electrophoresis the separated RNA fragments were transferred to a Hybond™-N⁺ membrane (Amersham) by capillary blotting according to Sambrook *et al*. (1989) Molecular Cloning: A Laboratory Manual, second edition, Cold Spring Harbor Laboratory Press, Cold Spring Harbor, New York, using 0.025 M phospate buffer, pH 7, as a blotting solution. After RNA transfer, the membrane was dried and the RNA cross linked to the membrane by UV treatment (312 nm, 0.6 J/cm²). The protocol for hybridisation of Northern blots was identical to that of hybridistation of DNA blots described before.

Complete cDNA fragments were obtained using the Superscript™ One-Step RT-PCR System (Life Technolgies).Therefore, total RNA was isolated from mycelium, in which *Bclcc*2 expression had been induced for three hours with tannic acid as described above, using the TRIzol protocol. Of this total RNA, 0.01 µg was reverse transcribed and amplified using the gene specific primers Lcc2-cDNA(+1)for (5'CATGAAGTATTCTACAGTCTTTA CTGCCCTCACTG-3') and Lcc2-cDNA(+1870)rev (5'-CCCTTAGATTCCAGAA TCGTCCTCGGCG-3'). The cDNA was directly cloned into the vector pCR® 4-TOPO® (Invitrogen) and completely sequenced.

### Example 3: Generation of Botrytis cinerea Bclcc1 and Bclcc2 disruption mutant.

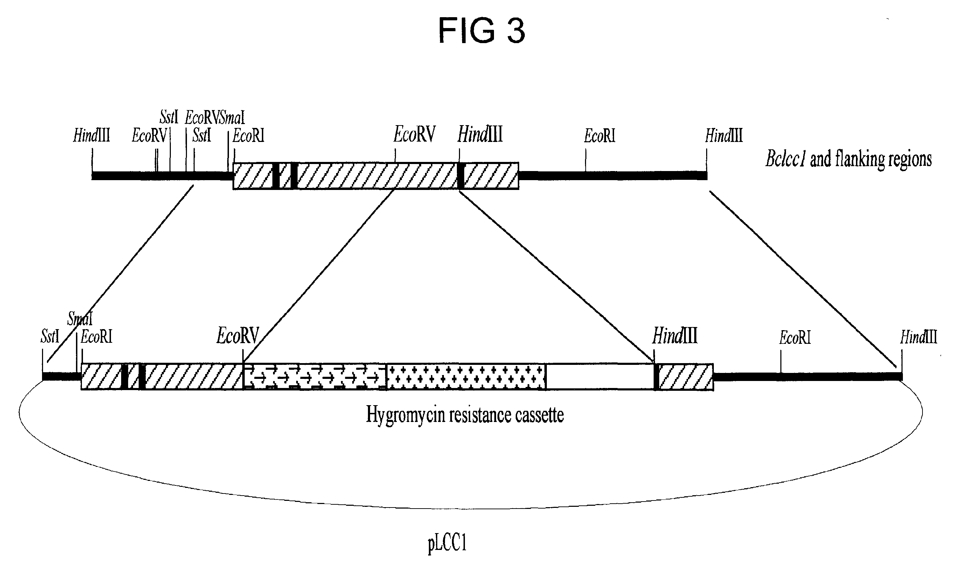

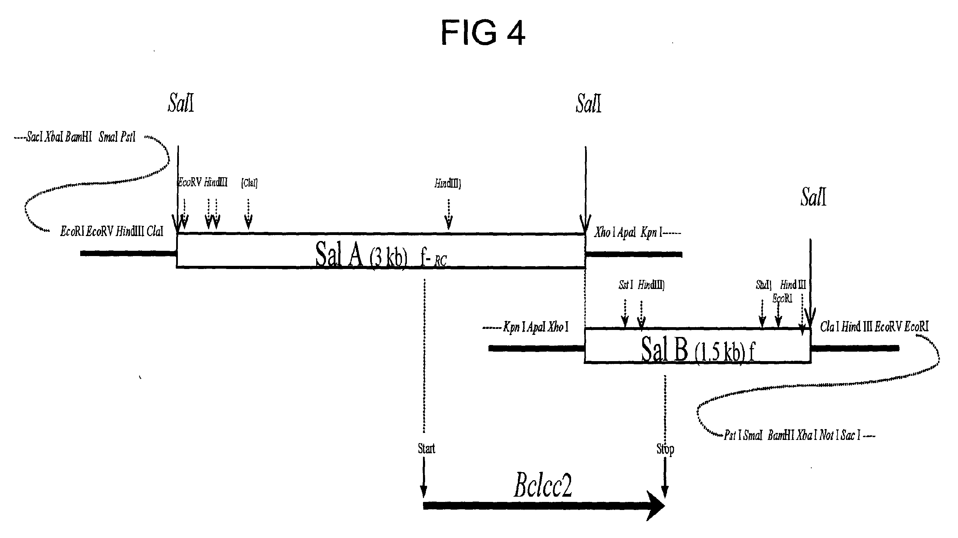

Transformation of *Botrytis cinerea* was performed with a vector for homologous recombination. To construct the vector for *Bclcc*1 gene disruption, the 1.1 kb *Sst*1*-Eco*RV fragment and the 1.5 kb *Hin*dIII fragment were ligated into vector pOHT as described by van Kan *et al.* (1997) Molecular plant-Microbe Interactions 10, 30-38 on both sides of the hygromycin resistance cassette (see figure 3). To construct the vector for *Bclcc*2 gene disruption, the complete *Bclcc*2 gene including flanking sequences was located on two *Sal*I fragments, which were both cloned in pBluescript (see figure 4). The SalA fragment contained the promoter region and the first 1300 bp of the gene. The SalB fragment contained the remaining part of the gene, including stop codon, followed by the terminator. From the SalA fragment a *Hin*dIII fragment was isolated and cloned in the *Hin*dIII site of the transformation vector pOHT (see van Kan *et al*., (1997) Molecular plant-Microbe Interactions 10, 30-38), resulting in the vector pLCC2-H. From the vector containing the SalB fragment a *Sst*I fragment was isolated and cloned in the *Sst*I site of the transformation vector pLCC2-H, resulting in the vector pLCC2-HS. When compared to the hygromycin selection marker, the fragments of both clones were oriented anti parallel in pLCC2-HS.

To obtain protoplasts for transformation, 100 ml 1% (w/v) Malt Extract (Oxoid) was inoculated with 2×10⁷ *Botrytis cinerea* strain B05.10 conidia. After 2 hours, the germinating conidia were incubated at 20°C on a rotary shaker at 180 rpm for 24 hours. The mycelium was harvested using a 22.4 µm sieve and incubated in 50 ml KC solution, containing 0.6 M KCl and 50 mM CaCl₂, supplemented with 5 mg/ml novozyme. After protoplasting, the suspension was filtrated over a glass wool filter and subsequently sieved over a 22.4 µm and a 10 µm sieve. The protoplasts were washed and resuspended to a final concentration of 10⁷ protoplasts per 100 µl.

For gene disruption, 2 µg of transformation vector (pLCC2-HS or pLCC1) was diluted in 95 µl KC and 2 µl 50 mM spermidin was added. After incubation on ice for 5 min, 100 µl protoplast suspension was added to the DNA and further incubated on ice for 5 min. Next, 100 µl polyethylene glycol (PEG) solution, containing 25% (v/v) PEG 3350 in 10 mM Tris-HCl, pH 7.4, and 50 mM CaCl₂, was added and the suspension was mixed. After 20 min at room temperature, again 500 µl PEG was added and the tubes were left at room temperature for another 10 min. Finally, 200 µl KC solution was added. The transformation mixture with the transformed protoplasts was mixed with 500 ml SH agar and dispensed immediately over 20 petridishes. SH agar contains 0.6 M saccharose, 5 mM HEPES pH 6.5, 1.2% (w/v) purified agar and 1 mM NH₄(H₂)PO₄. After 24 hours incubation at 20°C, an equal volume of SH agar containing 50 µg/ml hygromycin was added. Individual emerging colonies were transferred to malt agar plates containing 100 µg/ml hygromycin for further selection. Growing colonies were transferred to malt agar plates without hygromycin and sporulation was induced by blacklight treatment. To obtain monosporeisolates, conidia were isolated, diluted and plated on malt agar plates containing 100 µg/ml hygromycin. Conidia obtained from these plates were isolated and used for further analysis. When not used immediately, the *Botrytis cinerea* disruption mutants isolated conidia were frozen at -80°C in 75% (v/v) glycerol containing 12 mM NaCl.

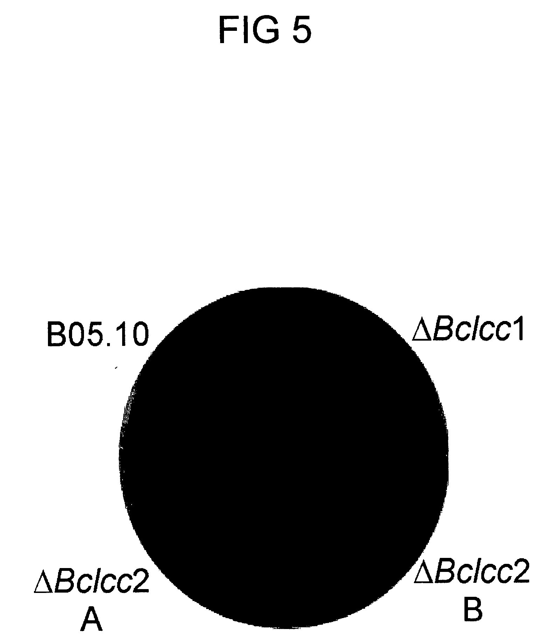

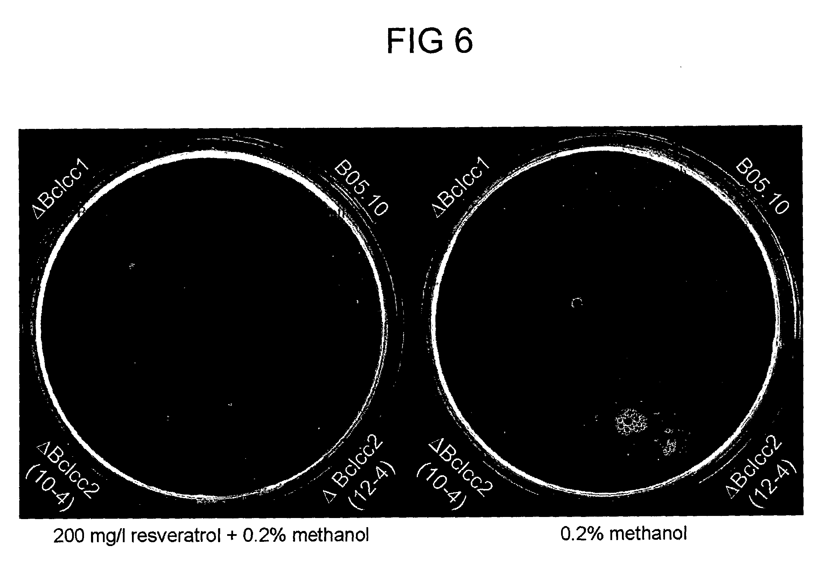

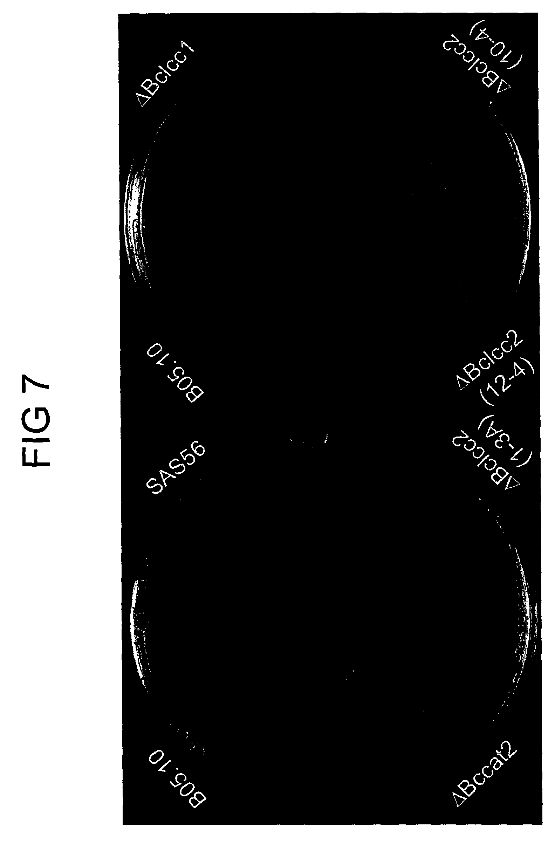

To detect putative *Bclcc1* and *Bclcc2* gene disruptants the tannic acid assay was performed (see figure 5). Transformants were tested on their ability to convert tannic acid into a dark brown pigment. Therefore *Bortytis cinerea* wild type B05.10 and putative *Bclcc*2 disruptants were grown on a basal medium, supplemented with CuSO₄ and tannic acid according to Kerssies (1990) Netherlands Journal of Plant Pathology 96, 247-250. The basal medium contained 11.8 mM NaNO₃, 6.9 mM K₂HPO₄, 0.81 mM MgSO₄.7H₂O, 2 mM KCl, 110 mM glucose and 25 g/l agar. This medium was sterilised and after cooling down a filter sterile solutions of tannic acid and CuSO₄ to final concentrations of *2.5* g/l and 3.4 mM, respectively, were added directly before pouring the plates. The plates were inoculated with a 2 µl droplet containing 10⁶ conidia/ml. As is depicted in figure 5, *Botrytis cinerea* wild type strain B05.10 is capable of converting tannic acid, added to agar medium, by a laccase or a laccase-like enzyme into a brown pigment (as described before by Kerssies (1990) Netherlands Journal of Plant Pathology 96, 247-250). All *Bclcc*2 disruptants have lost this capability of conversion. The *Bclcc*1 disruptants showed the same phenotype as the wildtype strain B05.10 with respect to tannic acid conversion. Apparently, BcLCC2 is the specific laccase to convert tannic acid. Additionally, *Botrytis cinerea* wild type B05.10 and putative *Bclcc1* and *Bclcc2* disruptants were also grown on plates containing resveratrol. As mentioned before, *Botrytis cinerea* laccase activity is thought to detoxify resveratrol. To determine whether or not the aforementioned laccase genes were involved in resveratrol degradation, both the wild type B05.10 and the *Bclcc*1 and *Bclcc*2 disruptants were tested for growth on agar plates containing resveratrol. The wild type strain B05.10 and the *Bclcc*1 disruptants were capable of converting resveratrol, resulting in a clear yellowish halo and yellow pigmented mycelium (see figure 6). The *Bclcc*2 disruptants did not show this phenotype. No halo formation was observed and the mycelium remained white. Prolonged growth showed that the *Bclcc*2 disruptants grew significantly better until they reached the halo produced by the wild type strain B05.10 or the *Bclcc*1 disruptant. Mycelium from the *Bclcc*2 disruptants reaching this halo grew slower and showed the same phenotype as the wild type strain B05.10 and the *Bclcc*1 disruptants, i.e. the mycelium became yellow pigmented (see figure 7). This demonstrates that resveratrol conversion resulting in fungitoxic compounds is caused by BcLCC2 activity and is not depending on plant derived enzymes.

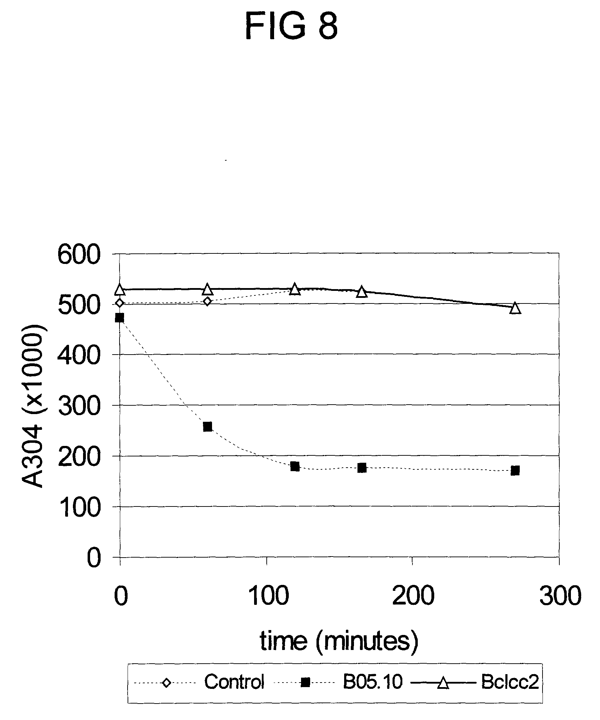

The laccase enzyme activity in the wild type strain B05.10 or the *Bclcc*2 disruptant was detected using the resveratrol assay mentioned before (see Pezet *et al.* (1991) Physiological and Molecular Plant Pathology 39, 441-450). *Bclcc*2 expression is induced by addition of the substrate resveratrol to the culture. Resveratrol has a UV absorption spectrum with a peak at 304 nm. Conversion products of resveratrol do not contain this absorption peak. A spectrophotometric assay using resveratrol showed that a culture filtrate obtained from an *in vitro* culture of *Botrytis cinerea* wild type strain BO5.10 was capable of rapidly converting resveratrol 19 hours after induction with resveratrol (see figure 8). This was not the case for a culture filtrate obtained from a *Bclcc*2 disruptant demonstrating that resveratrol induces *Bclcc*2 expression in wild type *Botrytis cinerea* and that, at this stage, BcLCC2 is solely responsible for resveratrol conversion.

The bioassay for comparison of the laccase mutants to wild type strain B05.10 can be performed on whole plants or detached leaves of *Vitis vinifera*, *Arachis hypogeae* or any plant capable of synthesising resveratrol. A useful procedure could be the following. Prior to inoculation the resveratrol synthesis is induced by UV-irradiation treatment according to Langcake and Pryce (1977) Phytochemistry 16, 1193-1196. To stimulate germination, *Botrytis cinerea* spores are preincubated in B5 medium, supplemented with 10 mM glucose and 10 mM (NH₄)H₂PO₄, for 2 hours at room temperature. The leaves are inoculated on the upper side with drops of 2 µl containing 500 spores. Inoculated leaves are incubated at more than 95% humidity at various temperatures, ranging from 4°C to 20°C, both in the dark and in combination with various light conditions. Emerging primary lesions are counted and expansion of the lesions is followed in time.

SEQ ID No. 1:
MKYSTVFTALTALFAQASATAIPAVRSPLAPRQSTTASCANSATSRSCWGEYSIDTNW
YDVTPNTGVTREYWLSVENSTITPDGYTRSAMTFNGTVPGPAITADWGDNLIIHVTNN
LQHNGTSIHWHGIRQLGSLEYDGVPGVTQCPIAPGDTLTYKFQATQYGTTWYHSHFSL
QYADGLFGPLIINGPATADYDEDVGAIFLQDWAHKSVFEIWDSARQGAPPALENTLMN
GTNIYDCSASTDANCVGGGKKFELTFVEGTKYRLRLINVGIDSHFEFAIDNHTLTVIA
NDLVPIVPYTTDTLLIGIGQRYDVIVEANAAADNYWIRGNWGTTCSSNSEAANATGIL
RYDSSSTVDPTSVGVTPRGTCADEPVASLVPHLALDVGGYSLVDEQVSFAFTNYFTWT
INSSSLLLDWSSPTTLKIFNNETIFPTDYNVVALNQTDANEEWVVYVIEDLTGFGIWH
PIHLHGHDFYVVAQETDVFSATKSPANFNLVNPPRRDVAALPGNGYLAIAFKLDNPGS
WLLHCHIAWHASEGLAMQFVESQSSIAIGMSDTDIFEDTCANWNAYTPTELFAEDDSG
I

## Claims

1. A recombinant protein having laccase activity, exhibiting at least 60% amino acid identity, as determined by the BLAST algorithm, with the amino acid sequence of SEQ ID No. 1 or a part thereof having at least 15 contiguous amino acids which are identical to the corresponding part of the amino acid sequence of SEQ ID No. 1.

2. A recombinant protein according to claim 1, exhibiting at least 70%, preferably at least 80%, amino acid identity with the amino acid sequence of SEQ ID No. 1.

3. A recombinant protein according to any one of the preceding claims which converts resveratrol into phytoalexins or tannic acid into a dark brown pigment.

4. A nucleotide sequence encoding a recombinant protein according to any one of the preceding claims.

5. A nucleic acid construct comprising the nucleic acid sequence of claim 4, operationally linked to an expression-regulating nucleic acid sequence.

6. A recombinant host cell containing one or more copies of the nucleic acid construct according to claim 5.

7. A recombinant host cell according to claim 6 containing one or more copies of a nucleic acid construct comprising one or more nucleic acid sequences encoding one or more recombinant proteins having resveratrol synthase activity.

8. A recombinant host cell according to claim 6 or 7 wherein said recombinant host cell is selected from a group consisting of mammalian (with the exception of human), animal, fungal, yeast or bacterial cells.

9. A recombinant host cell according to claim 6 or 7 wherein said recombinant host cell is a plant cell.

10. A process of producing a protein with laccase activity, comprising culturing a host cell according to any one of the claims 6-9 in a culture medium, and optionally recovering the protein from the culture medium or the cell free extract.

11. A process of producing phytoalexins of interest, using a protein according to any one of claims 1-3 or a host cell according to any one of the claims 6, 8 or 9 and resveratrol or a host cell according to any one of the claims 7-9.

12. A process of controlling animal and microbial pests in plants or plant cells by applying phytoalexins produced by a process according to claim 9.
